Europäisches Patentamt

European Patent Office

Office européen des brevets

(11) Publication number: **0 255 355 A2**

# (12) EUROPEAN PATENT APPLICATION

(21) Application number: **87306693.0**

(22) Date of filing: **29.07.87**

(51) Int. Cl.⁴: **A 01 H 1/02**

(30) Priority: **30.07.86 US 890528**

(43) Date of publication of application:
**03.02.88 Bulletin 88/05**

(84) Designated Contracting States: **DE FR GB SE**

(71) Applicant: **ALLELIX INC.**
**6850 Goreway Drive**
**Mississauga Ontario, L4V P1L (CA)**

(72) Inventor: **Beversdorf, Wally**
**116 Applewood Crescent**
**Guelph Ontario N1H6B6 (CA)**

**Pauls, Karl Peter**
**44 James Street West**
**Guelph Ontario N1H6B6 (CA)**

**Van Phan, Chuong**
**83 Conroy Crescent**
**Guelph Ontario N1H6B6 (CA)**

(74) Representative: **Hildyard, Edward Martin et al**
**Frank B. Dehn & Co. Imperial House 15-19 Kingsway**
**London WC2B 6UZ (GB)**

(54) **Haploid protoplast fusion.**

(57) This invention relates to a process for fusing protoplasts derived from haploid Brassica tissue, comprising the steps of
(A) obtaining haploid microspores from a plant grown from diploid Brassica seed;
(B) culturing said microspores to produce a culture;
(C) deriving from said culture a plurality of haploid protoplasts capable of fusion to form a regenerable diploid synkaryon; and
(D) inducing fusion among said plurality of haploid protoplasts to produce a regenerable diploid synkaryon, to regenerable diploid synkaryons produced by the process of the invention, and to plants and seeds derived therefrom.

**Description**

"Haploid protoplast fusion"

BACKGROUND OF THE INVENTION

The availability of haploid material can have a significant impact on plant improvement programs. The development of in vitro methods for generating haploids has resulted, for example, in a substantial saving of time for the early release of new varieties of certain crops. Since haploids possess only one set of alleles at each locus of the genome, recessive characters otherwise masked by the presence of a dominant gene are readily detectable via haploid culture. Moreover, the doubling of haploid genomes can be readily accomplished, either spontaneously or by the treatment of the haploid cells with colchicine, permitting the rapid production of a diploid homozygous line expressing hitherto masked characteristics. Haploids have thus found application in breeding programs to decrease the time required to produce homozygous lines and to increase the selection efficiency for genotypes combining recessive traits.

Another key aspect of many plant improvement programs is the availability of cytoplasmic male sterility (CMS) for use in producing hybrids, as disclosed in U.S. patent No. 4,517,763, the contents of which are hereby incorporated by reference. In oilseed rape, Brassica napus, several CMS systems have been developed, each classified on the basis of origin of the CMS trait, for example, "Napus-type," "Ogu-type" and "Polima-type." Each of the known B. napus CMS systems presents disadvantages, however. For example, there is no generally available maintainer line for Napus, while the CMS of Polima is unstable at higher temperatures. The source for Ogu-type CMS is radish cytoplasm, which can be transferred to rapeseed after sufficient crossing. But the CMS trait thereby transferred to rape is associated with a temperature-sensitive chlorophyll deficiency which results in chlorosis in the male-sterile lines at temperatures below 12°C.

The following literature citations provide background information on some of the existing CMS systems for B. napus and are hereby incorporated by reference:

Banneroot et al, Eucarpia Cruciferae Newsletter 2: 16 (1977)(Ogu-type CMS).

Hinata & Nonno, Japan. J. Breed 29: 305-11 (1979) (Diplotaxis Muralis-type CMS).

Thomas, Heredity 29: 253-57 (1972)(Napus-type CMS).

Sernyk, in PROC. BRASSICA WORKSHOP ON HYBRIDS & ANALYT. CHEM. 11 (Paris 1983)(Polima-type).

To overcome the problems of known CMS systems in Brassica, a somatic hybridization approach has been proposed (PCT application No. WO 84/03606) that involves the fusion of diploid (2n) plant cells called "protoplasts," from which the pectocellulose cell walls have been removed, either mechanically or by enzymatic digestion. More specifically, protoplasts derived from leaf tissue of normal (diploid) "parent" plants are fused, following the method of Chupeau et al, and the fusion products are cultured, first in a liquid nutritive medium and later on a solid agar nutrient substrate, to produce plantlets. The plantlets are grown into mature rape plants that can be screened for the CMS trait contributed by one parent and a cytoplasmic marker, such as triazine resistance, from the other, fertile parent. In this way, those cytoplasmic hybrids ("cybrids") produced by the disclosed fusion process that evidence an undesired contribution from the fertile parent, such as triazine-susceptibility, can be culled.

The above-cited PCT application does not differentiate between diploid products, on one hand, and the inevitable tetraploid (4n) and aneuploid ploidies of the disclosed fusion process; thus, no mention is made of a protocol for winnowing out the latter ploidies. In fact, tetraploids and aneuploids in Brassica generally display abnormal growth habits, aberrant flowers, poor seed set and partial female sterility. Accordingly, the transfer to rape of CMS via the disclosed diploid protoplast fusion process entails inefficiency in producing and then eliminating tetraploid and aneuploid by-products.

In theory, fusion of haploid (1n) protoplasts could be employed to avoid aberrant ploidies in regenerated plants. The fusion of haploid protoplasts is analogous to the fusion of gametes, but with the involvement of the male cytoplasm assured in the resulting cybrids. There is little reported work, however, where haploid protoplasts have been used in somatic hybridization. See Bajaj, "Haploid Protoplasts," in INT'L REV. CYTOL., Suppl. 16 (K.L. Girles ed. 1983), the contents of which are hereby incorporated by reference. When haploid protoplasts obtained from the anther-derived callus of Triticum aestivum and Oryza sativa were fused with mesophyll protoplasts of pea (Pisum sativa), cybrids revived after cryogenic manipulation only occasionally underwent first divisions, and in only one instance were repeated divisions observed. Id. Plants have been successfully regenerated from cultures derived from fusions between two haploid lines of N. plumbaginifolia, but regenerants were self-sterile and displayed aberrant morphology suggestive of unintended genetic changes. Sidorov and Maliga, Mol. Gen. Genet. 186: 328-32 (1982). There have been no published reports on the formation of somatic Brassica hybrids via fusion of haploid protoplasts.

SUMMARY OF THE INVENTION

It is therefore an object of the present invention to provide a method for the reliable production of Brassica cybrids or hybrids by culturing the fusion products of haploid protoplasts.

It is also an object of the present invention to provide diploid fusion products, obtained from haploid Brassica protoplasts, that can be used in plant breeding programs to exploit desired combinations of extranuclear traits, including cytoplasmic male sterility.

In accomplishing the foregoing objects, there has been provided, in accordance with one aspect of the present invention, a process for fusing protoplasts derived from haploid Brassica tissue, comprising the steps of

(A) obtaining haploid microspores from a plant grown from diploid Brassica seed;

(B) culturing said microspores to produce a culture;

(C) deriving from said culture a plurality of haploid protoplasts capable of fusion to form a regenerable diploid synkaryon; and

(D) inducing fusion among said plurality of haploid protoplasts to produce a regenerable diploid synkaryon.

In a preferred embodiment, the haploid microspores cultured in step (C) are produced from two different plants, each having at least one distinguishing marker, and the fusion of haploid protoplasts derived from culturing the haploid microspores yields a diploid product carrying a distinguishing marker from each of the plants.

There has also been provided, in accordance with another aspect of the present invention, a regenerable, diploid synkaryon of Brassica. In a preferred embodiment, the synkaryon of the present invention is both a cytoplasmic hybrid (cybrid) and a nuclear hybrid comprising material from two different sources.

Other objects, features and advantages of the present invention will become apparent from the following detailed description. It should be understood, however, that the detailed description and the specific examples, while indicating preferred embodiments of the invention, are given by way of illustration only, since various changes and modifications within the spirit and scope of the invention will become apparent to those skilled in the art from this detailed description.

## BRIEF DESCRIPTION OF THE DRAWINGS

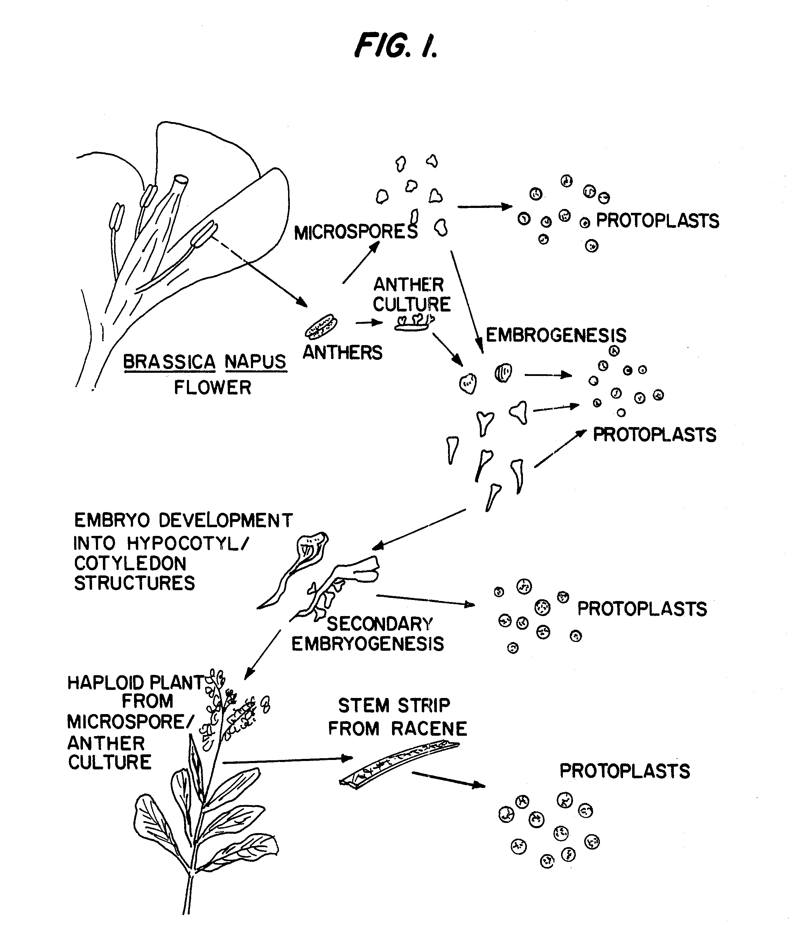

Figure 1 is a schematic illustration of various stages in Brassica microspore culturing from which materials for the production of haploid protoplasts used in accordance with the present invention can be obtained.

## DETAILED DESCRIPTION OF THE PREFERRED EMBODIMENTS

For the purposes of the present description, the term "Brassica" is used to denote crop plants in six major species as described, for example, by Yarnell, Bot. Rev. 22: 81 (1956). These include three diploid species, B. nigra (bb), B. campestris (aa) and B. oleracea (cc), that are the progenitors of naturally occurring allotetraploid species B. juncea (aabb), B. napus (aacc) and B. carinata (bbcc). Also included within "Brassica" are the numerous subspecies or varieties that comprise a range of forms resulting from divergent selection during domestication of the aforesaid species.

It has been discovered that somatic transfer of cytoplasmic traits in Brassica can be accomplished by fusion of protoplasts derived from haploid material at several stages in the development of mature haploid plants from cultured microspores (see Figure 1). In the illustrative examples below of the present invention, the protoplasts used for fusion were obtained from stem tissue of haploid B. napus, as mature plants are the preferred source of haploid protoplasts for use in the present invention. But suitable haploid protoplasts can be produced, in accordance with the present invention, directly from Brassica microspores, as well as from proembryos, embryos and embryogenic callus derived from such microspores. When the haploid protoplasts are fused, as described below, the diploid fusion product containing a single nucleus is called a "synkaryon."

A preferred source for microspores is anther culture, as disclosed by Chuong and Beversdorf, Plant Sci. 39: 219 (1985), and Lichter, Z. Pflanzenphysiol. 103: 229 (1981), the respective contents of which are hereby incorporated by reference. Generally, anthers obtained from flower buds of plants grown from diploid Brassica seed are macerated in a washing solution of B5 or other known medium, and the resulting suspension is aseptically filtered to obtain microspores. By another preferred method, whole Brassica flower buds are homogenized, at high speed, in a microblender of the type sold by Micro S/S Eberbach Co. (Ann Arbor, MI), which microblender contains cool (about 12°C) wash solution; filtering of the homogenate yields large numbers of microspores free of tissue and cellular debris.

The microspores are resuspended in a culture medium comprising conventional micro and macro elements, sugars, vitamins and growth-regulating factors like naphthalene-acetic acid (NAA) and gibberellic acid. Initiation of successful microspore culture, characterized by an increase in microspore volume, usually occurs within 24 hours at around 30°C. After about 5 to 7 days, cell clusters with well-defined epidermal layers (proembryos) are observed in culture, followed by the observation of typically heart-shaped or torpedo-shaped embryos within approximately two weeks. After some four weeks in culture, samples of embryos are transferred to a basal solid medium where embryogenic callus can develop. From a sizable percentage (usually 10% to 40%) of the calli, plantlets can be regenerated within about three weeks; approximately 80-85% of the plantlets are haploid, the remainder consisting of diploids with normal flowers and seed-setting behavior.

As previously mentioned, haploid protoplasts for fusion according to the present invention can be obtained from Brassica material at any of the above-described stages of microspore culture. Protoplasts can be isolated from such material by mechanical methods, for example, by gently tearing callus tissue apart in liquid culture medium with dissecting needles, but yields are generally low. Cell-wall removal by an enzymatic process is therefore preferred unless all side effects of wall-degrading enzymes must be avoided.

As described by Eriksson, "Protoplast Isolation and Culture," in PLANT PROTOPLASTS 1-20 (CRC Press

3

1983) (hereafter "Eriksson"), the contents of which are hereby incorporated by reference, the enzymatic isolation of protoplasts can be performed in two different ways: the two-step (or sequential method) and the one-step method. In the two-step method, the tissue is first treated with a macerozyme or pectinase which separates the cells by degrading the middle lamella. The cells thus freed are then treated with cellulase, which releases the protoplasts. In general, the cells are exposed to the different enzymes for shorter periods than are used for the one-step method. In the one-step method the tissue is subjected to a mixture of enzymes, including macerozyme and cellulase. This method generally results in higher yields from leaf tissues since both mesophyll and palisade cells release protoplasts. The one-step method, which is also less labor intensive, is therefore preferred. A number of cell wall-degrading enzymes suitable for use in accordance with the present invention are listed in Table 1.

## Table 1 *

### CELL WALL DEGRADING ENZYMES MOST COMMONLY EMPLOYED IN PROTOPLAST ISOLATION

| Enzyme | Manufacturer/Supplier |
|---|---|
| **Cellulases and hemicellulases** | |
| Cellulase | Calbiochem, La Jolla, Calif., U.S. |
| | Mayvill Chemicals Ltd., Cheshire, U.K. |
| | Serva Feinbiochemica, Heidelberg, W.Germany |
| | Sigma London Chemical Co, Dorset. U.K. |
| Cellulase Onozuka RIO | Yakult Honsha Co., Nishinomiya, Japan |
| Cellulase RID | Yakult Honsha Co., Nishinomiya, Japan |
| Cellulase RS | Yakult Honsha Co., Nishinomiya, Japan |
| Cellulysin | Calbiochem, La Jolla, Calif., U.S. |
| Driselase | Kyowa Hakko Kogyo, Tokyo, Japan |
| Hemicellulase | Sigma London Chemical Co., Dorset, U.K. |
| Meicelase | Meiji Seika Kaisha, Tokyo, Japan |
| Rhozyme\ HP150 | Genencor Inc., Corning, U.S. |
| **Pectinases** | |
| Macerozyme RID | Kiki Yakult, Nishinomiya, Japan |
| Macerase | Calbiochem LaJolla, Calif., U.S. |
| Pectinase | Sigma Chemicals, St. Louis, U.S. |
| Pectinol R-10 | Rohm and Haas, Philadelphia, U.S. |
| Pectolyase Y23 | Sigma London Chemical Co., Dorset, U.K. |
| Rohament P | Rohm GmbH, Darmstadt, W.Germany |
| **Other mixtures** | |
| Glusulase | Endo Laboratories, New York, U.S. |
| Helicase | Industrie Biologique Francaise, Gennevilliers, France |

* Modified from Eriksson (1983).

Since protoplasts are negatively charged and will not spontaneously fuse at usable frequencies, fusion of the protoplasts has to be induced. The preferred agent for the agglutination and fusion of haploid Brassica protoplasts according to the present invention is polyethylene glycol (PEG), employed as a solution of PEG 1540, 4000 or 6000 at a final concentration of 25% to 30%. The frequency of fusion can vary, usually involving between about 10% and 40% of the protoplasts treated, primarily as a function of the density of the protoplast preparation, the duration of treatment with PEG (10 to 40 minutes, preferably 30 to 35 minutes), and pH. Fusion frequency can be increased by enrichment of the treatment solution with $Ca^{2+}$ and/or by increasing pH to between about 9.0 and 10.5.

Other chemical methods, such as disclosed by Lazar, "Recent Developments in Plant Protoplast Fusion and Selection Technology," in PROTOPLASTS 1983 LECTURE PROCEEDINGS (I. Potrykus, C. T. Harrus, A. Hinnen, R. Hutter, P. J. King & R. D. Shillito) (hereafter "Lazar"), the contents of which are hereby incorporated by reference, can be used to induce protoplast fusion in the present invention. But the PEG method is preferred by virtue of its higher efficiency and the ready availability of PEG.

Electrical methods can also be used to induce protoplast fusion, but such methods require specialized equipment. For example, fusion of protoplasts can be induced using an electric field pulse technique disclosed by Vienken et al, Physiol. Plant. 53: 64 (1981). The two-step procedure entails the application of an alternating, nonuniform electric field to protoplast suspensions. With dielectrophoretic collectors adjusted to 1.5 V and 1 MHz, and with the electrical conductivity of the suspension medium at around $10^{-5}$ S/cm or less an electrophoresis effect is generated which makes the cells attach to each other along the field lines. Injection of an electric field pulse of high intensity (typically 750 to 1000 V/cm) for a short duration (about 20 to 50 sec) then leads to breakdown of membranes and fusions.

When cultured in one of the many suitable media available to the art, for example, as disclosed in PLANT TISSUE CULTURE METHODS No. 19876 (Nat'l Res. Council Canada 1982), the fusion products produced in accordance with the present invention behave like nonfused protoplasts. Within a day or two, fusion products typically acquire an oval shape indicative of cell wall formation, and viable fusion products will show cyclosis. Fusion products can be separated from the unfused parental cells by a variety of methods as described by Lazar. In particular, the partners used for fusion can be selected to have complementary deficiencies that either are genetic (such as chlorophyll deficiency) or are induced by treating one or more of the protoplast populations with a metabolic inhibitor (e.g. sodium iodoacetate). Among fusions between such protoplasts, only synkaryons will survive. Alternatively, physical properties such as cell size, buoyant density, color and fluorescence can be used to distinguish the fusion products in a fusion mixture from the unfused parents.

Synkaryons can be isolated after protoplast fusion on the basis of these criteria, manually with a micropipette, by centrifugation in a density gradient, by electrophoresis or by flow cytometry/cell sorting. Flow cytometry is an automated technique which can be used to quantify single cell characteristics such as cell size, granularity, DNA content and fluorescence intensity and on the basis of the measurements, to select subpopulations of similar cells from a mixture. The advantages of this technique are its speed and general utility. With appropriate fluorescent tags, the technique can be applied to protoplasts derived from plant material of any origin, thereby avoiding extensive preselection trials for plants with selectable traits. In addition, the availability of DNA-specific fluorochromes, including Hoechst dyes 33258 and 33342, propidiumiodide, ethidiumbromide and mithramycin, permit qualification of DNA in fusion products by flow cytometry or microspectrophotometry, and, thereby, the ready identification of diploid cybrids produced in accordance with the present invention.

Phenotypic markers distinguishing the parental lines, particularly cytoplasmic male sterility (CMS) and triazine resistance (TR), can also be used to categorize post-fusion regenerants, based on the presence or absence of the respective markers. This method for selection of hybrids is particularly useful if no cell sorter and no mutant lines are available. Otherwise, the choice of selection method is not crucial. Regenerants can also be screened for ploidy, and the diploids selected, as illustrated in greater detail below.

In addition to the methodology and media described below for isolating protoplasts and recovering plants from the synkaryon of the present invention, other protocols developed for application to B. napus material can be used to regenerate plants from protoplasts fused in accordance with the present invention. The following literature citations disclose several such protocols, and are hereby incorporated by reference:

Barsby et al, Plant Cell Reports 5 : 101-03 (1986).
Spangenberg et al, Physiol. Plant 66 : 1-8 (1986).
Chuong et al, Plant Cell Reports 4 : 4-6 (1985).
Glimelius, Physiol. Plant 61 : 38-44 (1984).
Kohlenbach, Z. Pflanzenphysiol. 105 : 131-42 (1982).

The present invention will be further described by reference to the following illustrative examples.

Example 1. Embryogenesis in cytoplasmically male sterile plants of B. napus via microspore culture.

Cytoplasmic male sterile (CMS) B. napus lines of the Polima-type (Pol-CMS) were maintained by successive backcrossing to the B. napus cultivars "Regent," "Altex" and/or "Topas" (as pollen parents). Seeds were sown in pots containing Metro-mix 245 (Grace & Co. of Canada Ltd., Ajax, Ontario) and plants were kept under 16 h photoperiod with a fluorescent illumination intensity of 200 lux/$m^2$ at the bench level. Plants were watered and fertilized daily. To determine the frequency at which normal pollen was produced by anthers of CMS plants, three anthers from three flowers were collected, macerated in acetocarmine solution and examined microscopically. Pollen which had a regular appearance and took up the stain uniformly was considered normal. To examine the nuclei in pollen produced by the CMS lines, the pollen was collected at anthesis, and prepared by hydrolyzing in 5N HC1 for 15 minutes, washed with distilled water, and macerating in 1 drop of 0.05% Toluidine blue in McIlvaine buffer (0.1 M citric acid and 0.2 M $Na_2HPO_4$).

Microspores for culture were isolated from anthers of several young buds harvested from main racemes and lateral branches of the donor plants. Microspores were extracted aseptically from anthers and cultured in modified Nitsch and Nitsch medium as described in Chuong and Beversdorf (1985), cited above, with 0.5 mg/l of NAA instead of 1 mg/1. Prior to plating, the densities of the microspore cultures were determined by counting with a hematocytometer. The culture plates were incubated at a constant 30°C or under a split temperature regime consisting of 3 days at 32°C followed by maintenance at 25°C. Both cultures were kept in the dark. After 4 weeks, the total number of embryos produced were recorded.

To induce plantlet development, the embryos were subcultured onto B5 medium (1) supplemented with 0.1% gibberellic acid and solidified with 0.6% agar. Embryos from plates in which more than 1000 developed were often quite small. These were divided between 2 or 3 plates in additional medium and incubated at the original temperature for another 10 days, to enhance embryo development, before they were transferred to the above-described, agar-solidified B5 medium. The number of plantlets that developed directly from the embryos after 1 month on the regeneration medium was recorded. The plantlets were placed into peat pellets and kept in closed containers until the roots were well developed. After acclimatization, the plants were grown to maturity in the growth room. At the time of flowering, each plant derived from Pol-CMS microspores was pollinated with Regent or Altex pollen to determine their seed setting behavior. Flower morphology, pollen fertility and seed setting behavior were used as criteria to estimate the ploidy of the regenerants.

## (A) Pollen fertility and classification of CMS lines

The flowers of Pol-CMS lines are characterized by wrinkled sepals, wavy petals and severely reduced arrow-shaped anthers. Approximately 90% of the Pol-CMS lines had flowers that had some anthers on which small, pocket-like, lobes containing pollen developed. More than 90% of the pollen in these pockets stained normally with acetocarmine. In addition, all of the stained pollen of the CMS lines was cytologically normal. The microspores from Pol-CMS plants had a normal uninucleate stage and normal pollen, having three nuclei at anthesis, which confirmed that the microspores were functional and suitable for culture.

## (B) Embryo development

Microspores from Pol-CMS plants responded to both the continuous (30°C) or the split-temperature (32°C/25°C) culture regimes by developing into somatic embryos. First divisions in the microspore cells were observed 3 days after initiation of the cultures but cultures at 32°C generally had larger numbers of microspores dividing by this time than those kept at 30°C. The first proembryos that had a well-defined epidermal layer were sometimes visible without magnification in cultures at 30°C after 7 days. Heart-shaped embryos were apparent at day 14 in cultures from both CMS lines maintained at 30°C Along with the normal proembryos and heart-shaped embryos, many abnormal structures also developed. Four weeks after the plating, about 25% of the embryos that developed from Pol-CMS microspores were normal in appearance, including torpedo-shaped and rudimentary cotyledonous embryos.

The microspore cultures yielded large numbers of embryos. Embryo production varied from plant to plant, but from 0.7 to 1.8% of the Pol-CMS microspores (average of 1.3%) developed into embryos, corresponding to 1 to 23 embryos per anther (Table 2). The maturation of Pol-CMS embryos, which developed in cultures where large numbers of embryos were initiated, was enhanced by adding more media to the culture and dividing it between several plates.

Table 2

Embryo production by microspore culture
initiated from Polima CMS lines cultured at 30°C

5 –

| Lines | Anther equivalents/plate | Microspores /anther | Total # of embryos produced | Embryos produced/ anther equivalents § | Embryos /microspores (%) |
|---|---|---|---|---|---|
| Pol 5 | 48 | 1352 | 1115 | 23.2 | 1.8 |
| Pol 20 | 60 | 583 | 244 | 4.1 | 0.7 |
| Pol x Alt | 60 | nd* | 35 | 0.6 | nd |
| (Pol x Reg)-1 | 90 | nd | 425 | 4.7 | nd |
| (Pol x Reg)-2 | 48 | nd | 189 | 3.9 | nd |
| (Pol x Top) | 90 | nd | 871 | 9.7 | nd |

* not determined

§ anther equivalent = all the microspores from one anther

(C) Ploidy of regenerants

15% of the Pol-CMS embryos germinated and developed into plantlets after the initial subculture, i.e., when the embryos were transferred from the microspore-culture medium to the regeneration medium. Embryos which failed to develop into plantlets grew abnormally and produced large masses of tissue. During the second and third subcultures, when the microspore- derived embryos were transferred to fresh regeneration medium, shoot development was induced. Secondary embryogenesis was also observed on tissue that developed from the primary embryos in later subcultures. About twenty percent of the plants derived from microspores were spontaneous double haploids (Table 3). None of the double haploid regenerants set seed without hand pollination.

Table 3

Percent of haploids and spontaneous double haploids
derived from microspore cultures of Pol CMS lines

| Lines | Temp. regime | Anther equivalents /plate | Microspores / anther | Plating density microspore /ml | Total # of embryos produced | Embryos /anther equivalents | Embryos /microspores (%) |
|-------|------|------|------|------|------|------|------|
| Diplo 5 | 32/25°C | 36 | 1417 | 20,000 | 720 | 20.0 | 1.4 |
| Diplo 12 | 32/25°C | 36 | 1581 | 23,000 | 605 | 16.8 | 1.1 |
| Diplo 15 | 32/25°C | 18 | 1385 | 10,000 | 252 | 14.0 | 1.0 |
| Diplo 16 | 32/25°C | 36 | 875 | 13,000 | 89 | 2.5 | 0.3 |
| Diplo 10 | 32/25°C | 18 | 543 | 4,000 | 2 | 0.1 | 0.0 |
| Diplo 20 | 30°C | 90 | 1523 | 55,000 | 2918 | 32.1 | 2.1 |
| Diplo 5 | 30°C | 40 | 1232 | 20,000 | 992 | 24.8 | 2.0 |

Example 2. Somatic transfer of cytoplasmic traits in B. napus by haploid protoplast fusion.

Stem peelings from microspore-derived haploid plants, produced in accordance with Example 1, of both triazine-resistant (TR) and cytoplasmic male sterile (CMS) B. napus lines were incubated for 16 hours, at 25°C, in an enzyme mixture containing 1.5% Cellulase Onozuka RIO, 0.3% Macerase, 10% mannitol and CPW salt(2) as described by Xu et al, Plant Sci. Lett. 24: 117 (1982). Incubation was effected in darkness on a shaker rotating at 80 rpm. The protoplasts thus obtained were freed of enzyme by three centrifugations (100 × g; 3 minutes) with fresh CPW salt solution containing 10% mannitol.

The washed protoplasts from pairs of TR and CMS lines were mixed in 0.5 ml of 9% mannitol solution, to which was then added 0.5 ml of PEG solution containing 9% mannitol, 30 mM $CaCl2•H_2O$, and 30% PEG 6000. After gentle agitation, the mixture was allowed to stand for between 10 and 15 minutes. Over the next 15 minutes, at intervals of about 5 minutes, three 0.5 ml aliquots of 9% mannitol where then added, respectively, with gentle agitation accompanying each addition. After 2 ml of CPW salt solution with 10% mannitol were added, the mixture was spun at 100 × g for 3 minutes to pellet the protoplasts. The pelleted protoplasts were resuspended in 5 ml of the CPW salt/10% mannitol solution, spun again at 100 × g for 3 minutes, and resuspended in VN culture medium.(3)

The protoplasts produced in this fashion were plated at a density of approximately $10^5$/ml of VN medium (2.5 ml/plate), and the plates were incubated in the dark at 25°C for about 10 to 16 days. Cultures containing dividing cells were each embedded in 2.5 ml of embedding medium(4) at 40°C. The embedded cultures were gradually exposed to light (2000 lux) delivered on a 16-hour/8-hour cycle of light and darkness. Within two to three weeks after embedding, the mixture of microcolonies which had developed in situ was spread onto 2N callusing medium.(5)Three to four weeks later, separate calli had developed that were transferred to BA1(6) or fresh 2N media for regeneration.

About 10% to 40% of the calli regenerated plantlets, depending on the genetic background of the protoplasts used for fusion. Flower morphology, seed-setting behavior and microspectrophotometric measurements of DNA content were used as indicators of ploidy in the regenerates. Root tips from regenerants were fixed with ethanol:acetic acid (3:1) at 4°C for 24 hr, hydrolyzed with 5N HC1 for 45 min at room temperature, stained in Feulgen for 1 hour, and then squashed in 45% acetic acid. DNA content stained by Feulgen in root tips was measured with a Zeiss MPV3 microspectrophotometer set at 560 nm. Only metaphase, anaphase and telaphase figures of mitosis were measured, and the values were compared to those obtained from plants of known ploidy level.

Atrazine resistance was determined by application of an atrazine solution to leaf blades and, in addition, by measuring the fluorescence intensity of chloroplasts in the regenerated plants. Cytoplasmic atrazine resistance is characterized by increased chlorophyll fluorescence because the resistance-imparting mutation, which affects the binding site for atrazine on a 32 kd protein in the chloroplast electron transport chain, also reduces photosynthetic efficiency, as disclosed by Ali and Souza-Machodo, Weed Res. 21: 191-97 (1981).

Plants regenerated from fusion of the Brassica protoplasts were diploids, (47%), and haploids (49%), the remainder being tetraploids (4%). Among the diploids, two types of cybrids -- CMS/TR and male-fertile/triazine sensitive -- were obtained.

Formulation of Media in Examples 1 and 2:

(1) For formulation of $B^5$ medium, see Garnborg et al, Exp. Cell Res. 50:151-158 (1968).

(2) CPW salts: 27.2 mg/l $KH_2PO_4$; 101 mg/l $KNO_3$; 1480 mg/l $CaCl_2 \bullet 2H_2O$; 240 mg/l $MgSO_4 \bullet 7H_2O$; 0.16 mg/l KI; 0.025 mg/l $CuSO_4.5 H_2O$.

(3) VN medium: basic ingredients of MS medium[1] plus 800 mg/l glutamine; 30 mg/l glutathione; 100 mg/l L-serine; 1 mg/l 2,4-D; 0.5 mg/l BA; 3% sucrose; 10% mannitol (pH = 5.7; filter sterile).

(4) Embedding medium: basic ingredients of MS medium[1] plus 0.625 mg/l BA; 0.625 mg/l 2-iP; 0.625 mg/l kinetin; 0.625 mg/l zeatin; 0.5 mg/l NAA; 5% mannitol and 0.6% agarose (medium autoclaved and cooled down to 40°C prior to embedding).

(5) Callusing medium 2N: embedding medium, but with 0.4% agarose instead of 0.6%, plus 200 mg/l casein hydrolysate and 1.5% sucrose.

(6) Regeneration medium BA1: basic ingredients of MS medium[1] plus 200 mg/l casein hydrolysate; 5 mg/l BA; 0.5 mg/l NAA; 1.5% sucrose and 0.4% agarose (pH 5.7).

## Claims

1. A process for fusing protoplasts derived from haploid Brassica tissue, comprising the steps of
(A) obtaining haploid microspores from a plant grown from diploid Brassica seed;
(B) culturing said microspores to produce a culture;
(C) deriving from said culture a plurality of haploid protoplasts capable of fusion to form a regenerable diploid synkaryon; and
(D) inducing fusion among said plurality of haploid protoplasts to produce a regenerable diploid synkaryon.

2. A process according to Claim 1, wherein step (A) comprises (i) macerating anthers or whole flower buds from said plant in a washing solution to produce homogenate and (ii) filtering said homogenate to isolate said haploid microspores.

3. A process according to Claim 1 or Claim 2, comprising the production of haploid microspores from two different plants, each of said plants having at least one distinguishing marker, and the fusion of haploid protoplasts derived from culturing of said haploid microspores such that said synkaryon carries a distinguishing marker from each of said plants.

4. A process according to Claim 3, wherein said distinguishing marker of each of said plants is selected from cytoplasmic male sterility and cytoplasmic herbicide tolerance.

5. A process according to any of the preceding Claims wherein step (C) comprises producing haploid protoplasts from proembryo tissue obtained from said culture.

6. A process according to any of Claims 1 to 4, wherein step (C) comprises producing haploid protoplasts from embryo tissue obtained from said culture.

7. A process according to any of Claims 1 to 4, wherein step (C) comprises producing haploid protoplasts from embryogenic callus tissue obtained from said culture.

8. A process according to any of Claims 1 to 4, wherein step (C) comprises regenerating haploid plants from said culture and producing haploid protoplasts from tissue obtained from said haploid plants.

9. A process according to Claim 8, wherein said tissue consists essentially of stem peelings from said haploid plants.

10. A process according to any of the preceding Claims, wherein said seed in B. napus seed.

11. A regenerable, diploid synkaryon of Brassica.

12. A synkaryon according to Claim 11, said synkaryon being a cybrid.

13. A synkaryon according to Claim 12, wherein said cybrid comprises nuclear genetic material from two different sources.

14. A synkaryon according to any of Claims 11 to 13, said synkaryon being the product of a process as claimed in any of Claims 1 to 10.

15. A plant of the genus Brassica regenerated from a synkaryon according to any of Claims 11 to 14.

16. Seed or other propagule of the plant according to Claim 15.

[1] For MS medium, see Eriksson (1983) at page 12 (Table 2).

0255355

# FIG. I.

MICROSPORES

PROTOPLASTS

ANTHER CULTURE

ANTHERS

BRASSICA NAPUS FLOWER

EMBROGENESIS

PROTOPLASTS

EMBRYO DEVELOPMENT INTO HYPOCOTYL/ COTYLEDON STRUCTURES

SECONDARY EMBRYOGENESIS

PROTOPLASTS

HAPLOID PLANT FROM MICROSPORE/ ANTHER CULTURE

STEM STRIP FROM RACENE

PROTOPLASTS